# FASCICULE DE BREVET EUROPEEN

(11) **EP 0 689 833 B1**
(45) Date de publication et mention de la délivrance du brevet: **23.12.1998**
(21) Numéro de dépôt: 95440032.1
(22) Date de dépôt: 28.06.1995
(51) Int. Cl.: A61K 9/14, A61K 9/16

(54) **Procédé de préparation de sphéroides d'actifs naturels, notamment d'origine végétale, et sphéroides ainsi obtenus**
Verfahren zur Herstellung von Naturwirkstoffe enthaltenden Spheroiden, insbesondere pflanzlichen Ursprungs, und so erhaltene Spheroide
Process for preparing natural actives containing spheroids, especially of vegetable origin, and spheroids thus obtained

(30) Priorité: 28.06.1994 FR 9408358
(43) Date de publication de la demande: 03.01.1996
(73) Titulaire: Jacob, Maurice, 34000 Montpellier (FR); Bataille, Bernard, 34980 St Gely du Fesc (FR); Jacob, Olivier, 34090 Montpellier (FR); Iderne, Michel, 67100 Strasbourg (FR)
(72) Inventeur: Jacob, Maurice, 34000 Montpellier (FR); Bataille, Bernard, 34980 St Gely du Fesc (FR); Jacob, Olivier, 34090 Montpellier (FR); Iderne, Michel, 67100 Strasbourg (FR)
(74) Mandataire: Metz, Paul

(56) Documents cités:
- EP-A- 0 539 304
- WO-A-94/08567
- FR-A- 2 364 059
- FR-A- 2 705 677

## Description

L'invention concerne des compositions de substances d'origine naturelle, telles que des végétaux, des oligo-éléments, des minéraux, des vitamines des composants animaux sous la forme de sphéroïdes et plus particulièrement la formulation de formes galéniques correspondant à des sphéroïdes médicamenteux, diététiques ou alimentaires, préparés avec des solutions en particulier d'origine végétale.

L'invention concerne également le mode de préparation de ces sphéroïdes à partir d'un procédé de micro-extrusion et de sphéronisation.

Elle concerne également les applications qui en découlent pour l'administration par voie orale humaine et animale des sphéroïdes qui peuvent contenir jusqu'à 30 % de composants naturels hydro-alcoolo solubles.

Il est connu de préparer des extraits de produits végétaux sous forme de solutions végétales extractives obtenues par macération, infusion, décoction, digestion, lixiviation.

L'utilisation de ces extraits liquides présente quelques inconvénients notamment quant à leur instabilité physique et chimique au cours de la conservation, quant à la faible teneur en constituants végétaux caractéristiques ou quant à la présence fréquente d'alcool éthylique en quantité plus ou moins importante ce qui n'est généralement pas souhaité pour l'administration par voie orale de produits notamment lorsqu'il s'agit de produits médicamenteux.

A ce jour, pour transformer de tels extraits en extraits secs susceptibles de limiter les inconvénients indiqués, on fait appel à des procédés mettant en oeuvre un apport calorifique important, tels que la nébulisation, le séchage sur cylindres rotatifs, l'évaporation sous pression réduite.

Si ces procédés permettent d'obtenir des composés végétaux secs sous forme de poudres plus faciles à utiliser, notamment administrables par voie orale, en revanche, ils font appel à une élévation de température qui peut altérer les constituants caractéristiques des végétaux concernés. De plus ces extraits secs sont très souvent hygroscopiques, ce qui conduit par reprise d'humidité à du mottage, à des risques de modification de la stabilité physique et chimique, à des difficultés de reproductibilité et de manipulation.

Dans les documents FR 2 682 874 et EP- 539 304 la préparation d'un extrait de principes actifs sous forme sèche adsorbable, notamment d'origine végétale, est réalisée. Cette préparation consiste d'une part à préparer un extrait en milieu liquide par un procédé classique, puis à déposer cet extrait sur des grains d'un support adsorbant, d'autre part à faire adsorber ces grains préalablement calibrés dans et sur des microgranules poreuses d'excipient nébulise.

Dans un tel cas, l'utilisation implique un mélange de grains enrichis de principes actifs notamment végétaux, avec des grains poreux préalablement formés pour préparer et stabiliser les extraits de principes actifs.

Dans le document WO-94/08567 un procédé de préparation de pilules par granulation du mélange d'un principe actif avec un liquide et un agent anti-adhèrent, est réalisé. Puis, le granulé est extrudé et sphéronisé.

En ce qui concerne, par ailleurs, l'extrusion et la sphéronisation, elles constituent un ensemble technique relativement complexe utilisé notamment pour la préparation de produits pharmaceutiques sous la forme de sphères de petite dimension ou sphéroïdes (300 à 1.500 microns de diamètre moyen). Le procédé de fabrication consiste à granuler c'est-à-dire humidifier une masse pulvérulente à l'aide d'une liquide aqueux ou non aqueux, puis à extruder cette masse humide à travers une filière à orifices calibrés, puis à rendre sphérique, c'est-à-dire sphéroniser, le produit extrudé.

Pendant le processus d'extrusion, la masse humidifiée est tassée et transformée par étirement en filaments compacts de section généralement circulaire et calibrée appelés classiquement extrudats. Pour obtenir des sphéroïdes, on place ces extrudats dans un appareil cylindrique renfermant dans sa partie inférieure un disque tournant à des vitesses variables et contrôlées, appelé sphéroniseur. Sous l'effet de la force centrifuge exercée par la rotation contrôlée du disque tournant, les extrudats se fragmentent régulièrement, puis se transforment en sphères par un effet de roulage-liage.

On admet que, pour pouvoir subir ce processus, la masse humidifiée doit faire preuve d'un degré de plasticité spécifique et contrôlée afin de permettre au processus d'extrusion et de sphéronisation de se produire, c'est-à-dire de déformer dans un premier temps la masse humidifiée et de la transformer dans un second temps en extrudat, puis en sphéroïdes. L'obtention d'une masse convenablement humidifiée pour correspondre à la plasticité spécifique nécessaire au bon fonctionnement de l'extrudeur et à la production d'extrudats transformables en sphéroïdes, nécessite de choisir des substances généralement en poudre, ayant une capacité à adsorber et à absorber des quantités prédéterminées de liquides aqueux ou non aqueux d'humidification. Le rapport adéquat entre la quantité de liquide d'humidification et la quantité de poudre est à déterminer pour obtenir la masse plastique adaptée à l'opération d'extrusion, puis de sphéronisation.

De même, il a été constaté que la possibilité de transformer les extrudats en sphéroïdes de sphéricité homogène, de granulométrie régulière préalablement définie, dépend aussi des caractéristiques plastiques des extrudats donc des caractéristiques liées à l'opération de sphéronisation proprement dite c'est-à-dire à la vitesse et à la durée de rotation du disque tournant.

L'invention a pour objet l'établissement de formulations simplifiées qui permettent l'administration sous forme sèche de préparations liquides contenant des substances naturelles à des hommes ou des animaux. Elle vise en outre également un procédé utilisant l'extrusion et la sphéronisation, qui permette de préparer des sphéroïdes à caractéristiques techniques définies, en utilisant les formulations simplifiées tout en assurant une stabilité physique et chimique dans le temps des composants notamment végétaux utilisés, sans dégradation thermique.

En d'autres termes, l'invention vise également un ensemble formulation-procédé de préparation de sphéroïdes à base de solutions d'origine naturelle faciles à mettre en oeuvre, permettant d'obtenir des composants, par exemple, végétaux secs absorbables par voie orale sous forme de sphéroïdes stables dans le temps, plus faciles à contrôler et reproductibles.

Ces sphéroïdes sont présentés tels quels ou enrobés, en vrac, en distributeur doseur, ou en gélules, comprimés, sachets ou toutes formes destinées à cette voie d'administration.

L'un des buts de l'invention est d'utiliser les propriétés adsorbantes et absorbantes intrinsèques élevées vis à vis des liquides aqueux et non aqueux tels que l'eau ou les mélanges en toutes proportions d'eau et d'alcool éthylique en particulier, de substances utilisées seules ou en association, de type polymères naturels ou synthétiques. Ces substances peuvent correspondre à des matières premières utilisées en pharmacie, en diététique, en alimentation telles que les celluloses microcristallines, les celluloses microfines, les amidons, les amidons modifiés, les polysaccharides divers et d'une manière générale les matières premières présentant un pouvoir adsorbant et absorbant des liquides aqueux et non aqueux communiquant à la masse humidifiée des capacités plastiques nécessaires à la production des extrudats et des sphéroïdes par le procédé d'extrusion et de sphéronisation.

Une des caractéristique de l'invention est de pouvoir associer aux substances adsorbantes et absorbantes des substances différentes telles que des sucres simples à faible pouvoir énergétique, des substances minérales et organiques pouvant correspondre en particulier à du lactose, sorbitol, mannitol, malto-dextrines, carbonates, citrates. Ces substances constituent des substances auxiliaires.

Une association particulièrement retenue peut contenir une cellulose microcristalline pour 20 % à 80 % de la masse totale à humidifier, du lactose pour 80 % à 20 %, de la gélatine à pouvoir gélifiant variable ou de la polyvinylpyrrolidone ou un dérivé cellulosique (éthyl, méthyl, carboxy) pour moins de 10 %. L'usage dans le cadre de l'invention de ces substances auxiliaires est dépendant de la finalité d'emploi des sphéroïdes préparés, autrement dit selon que les composants naturels et végétaux supportés par les sphéroïdes sont libérables rapidement (moins de trente minutes) ou progressivement (généralement sur une durée supérieure à une heure) dans un milieu naturel ou artificiel.

Une autre caractéristique de l'invention est d'utiliser des substances auxiliaires pour que la texture des sphéroïdes présente une porosité de volume variable pouvant atteindre 100 mm³ par gramme de sphéroïdes, adaptée à la libération rapide ou progressive des composants naturels, par exemple végétaux dans un milieu naturel ou artificiel. L'association retenue dans le paragraphe précédant satisfait à un gradient de porosité définie.

Une autre caractéristique de l'invention est d'utiliser comme liquides d'humidification des solutions d'origine naturelle, par exemple végétale, ce qui correspond à des solutions aqueuses ou non aqueuses contenant des composants caractéristiques naturels. Il est particulièrement courant que le liquide soit un mélange en proportions variables d'eau et d'alcool éthylique, proportions en relation avec les caractéristiques de solubilité des composants naturels.

C'est ainsi que pour obtenir une masse de plasticité compatible avec la technique d'extrusion puis de sphéronisation, la masse de substance adsorbante et absorbante doit être humidifiée par une quantité de liquide comprise entre 50 et 250 % de la masse à humidifier. Il a été constaté qu'en dehors de ces valeurs limites la masse humidifiée ne présente pas les caractéristiques de plasticité requise pour le bon fonctionnement de l'extrudeur, soit que la masse est trop fluide et ne permet pas l'obtention des extrudats, soit que la masse est trop sèche entraînant une surchauffe de la masse ainsi que d'une partie de l'appareil pendant l'extrusion, ce qui entraîne un blocage dans le fonctionnement de l'appareil.

Une autre caractéristique de l'invention est que la plasticité requise pour l'extrusion puis le sphéronisation est dépendante du rapport existant entre la quantité de liquide d'humidification et la quantité de la masse à humidifier. C'est ainsi que l'invention définit pour l'utilisation notamment des celluloses microcristallines, que la quantité de liquide d'humidification représentée par une solution d'origine végétale est comprise entre 90 % et 150 % de la quantité des celluloses microcristallines utilisées.

Selon une autre caractéristique de l'invention le fort pouvoir adsorbant et absorbant des substances de type polymérique sert à retenir et à fixer dans et sur les structures physiques desdites substances, les composants des solutions d'origine naturelle dénommées classiquement extrait liquide, teinture végétale, solution extractives, solution d'extrait sec, teinture mère, alcoolature, alcoolat, solution de composant végétaux et plus globalement solutions végétales.

Ces substances de type polymérique du fait de leur pouvoir élevé d'adsorption et absorption des solutions d'origine naturelle peuvent ainsi servir de support à diverses compositions utilisables notamment chez l'homme ou l'animal par voie orale, à des fins thérapeutiques, diététiques, préventives ou alimentaires.

Un autre objet important de l'invention est de présenter ces compositions sous forme de sphéroïdes préparés par un procédé perfectionné d'extrusion et de sphéronisation. Cet ensemble est adapté dans ses caractéristiques techniques pour exploiter les formulations simplifiées permettant de préparer des sphéroïdes de sphéricité homogène, de granulométrie régulière préalablement définie, contenant en concentrations importantes des composants caractéristiques des produits naturels. L'adaptation des caractéristiques techniques conduit, d'une part à définir la filière et la vitesse d'extrusion, à maîtriser la température pendant l'extrusion, température qui ne dépasse pas trente degrés Celsius, d'autre part à définir la vitesse et la durée de la sphéronisation, pour exploiter les formulations simplifiées permettant de préparer des sphéroïdes conformes aux spécifications préalablement définies.

Une caractéristique particulière de l'invention consiste à préparer des extrudats puis des sphéroïdes de solutions végétales à partir d'un extrait liquide tel que défini par les Pharmacopées, en utilisant une masse de celluloses microcristallines sensiblement égale à la masse d'extrait liquide, La détermination de ce rapport permet de préparer des sphéroïdes de granulométrie comprise entre 350 microns et 1.000 microns, pour un rendement d'au moins 95 % de la masse des sphéroïdes produits. Dans ce cas, il est courant d'utiliser pour la filière de l'extrudeur comporte des orifices de 800 microns de diamètre d'ouverture et de 800 microns de longueur qui est aussi l'épaisseur de la filière. Il est essentiel pour les caractéristiques des sphéroïdes que le rapport entre le diamètre des orifices et leur longueur soit sensiblement égal à un. Dans ce cas, la vitesse d'extrusion est de 100 tours par minute pour un appareil extrudeur de type bivis GABLER ou tout autre. Le cycle de sphéronisation pour un appareil sphéroniseur de 60 cm de diamètre ou autre est d'une durée de dix minutes. Il est caractéristique de souligner que la durée de sphéronisation est décomposée en deux phases essentielles une à vitesse de rotation plus rapide, 740 t/mn par exemple, d'une durée de 90 % du temps, une terminale à la vitesse de rotation plus réduite, 500 t/mn par exemple, d'une durée complémentaire. Dans ce cas, les sphéroïdes résultants de sphéronisation sont séchées à une température ne dépassant pas quarante degrés Celsius.

En outre, l'invention permet également de préparer des sphéroïdes pouvant contenir des concentrations importantes de composants caractéristiques de végétaux. Selon une caractéristique de l'invention, c'est l'usage de substances à pouvoir adsorbant et absorbant élevé de liquide aqueux et non aqueux (un minimum de 100 % de liquide adsorbé et absorbé), en particulier de solutions végétales de type extrait liquide, qui assure une teneur élevée des composants caractéristiques végétaux sur et dans les sphéroïdes préparés. Ainsi une capsule dure en gélatine (gélule) de taille standard n° 1 peut contenir de 400 à 450 mg de sphéroïdes supportant les composants de 400 à 450 mg d'extrait liquide.

Il est notamment possible de préparer des sphéroïdes contenant au moins la même quantité de composants végétaux caractéristiques que ce que contient la même masse de plante sèche.

L'invention permet également de produire des sphéroïdes dépourvus de sucres énergétiques tels que le saccharose notamment, débarrassés de tout liquide et notamment d'alcool éthylique lorsque les solutions végétales ou autres utilisées en contiennent, en supportant tous les composants végétaux contenus dans les "solutions Végétales" utilisées pour la préparation des sphéroïdes. Cette caractéristique est obtenue sans élévation importante de la température qui ne dépasse pas 40°C en évitant une altération significative des composants végétaux caractéristiques.

Procédé de production industrielle de sphéroïdes absorbables par voie orale dans lequel on prépare un extrait liquide de principes actifs, on met en contact cet extrait liquide avec un support absorbant et on sèche caractérisé en ce que l'on :
. prépare à partir d'une ou de plusieurs matières premières naturelles contenant au moins un principe actif un extrait liquide, contenant au moins le principe actif que l'on veut isoler par extraction aqueuse, alcoolique ou hydroalcoolique ou mixte ;
. humidifie une substance absorbante et adsorbante ou un mélange de ce type de substances par l'extrait liquide de telle sorte que la quantité d'extrait liquide soit comprise entre 50 et 250 % de la masse à humidifier ;
. extrude cette masse humide à une température inférieure à 30°C à travers une microfilière dont le rapport entre le diamètre des orifices et leur profondeur est sensiblement égal à l'unité pour obtenir des extrudats filaires ;
. sphéronise les extrudats filaires pour obtenir des sphéroïdes ;
. sèche les sphéroïdes ainsi obtenus à une température inférieure à 40°C ;
. calibre les sphéroïdes secs.

Les exemples suivants permettent de mieux expliquer l'invention.

### EXEMPLE 1 :

On a préparé de la façon décrite ci-dessus une forme de sphéroïdes contenant 17,2 % de constituants hydro-alcoolo solubles d'HARPAGOPHYTON, correspondant à 100,0 parties de plante sèche et ayant la composition suivante.

Pour un lot de 5.000 g environ de sphéroïdes la formule comporte :

| Ingrédient | Quantité en % en poids |
|---|---|
| Extrait liquide d'HARPAGOPHYTON | 4.275,0 g |
| Cellulose microcristalline | 4.275,0 g |

### Remarque :

L'extrait liquide contient 17,2 % de composants hydro alcoolo solubles d'HARPAGOPHYTON. Son titre alcoolique est de 30 %. Sa teneur en harpagoside composant caractéristique est de 1,2 g pour 100 g d'extrait liquide.

### Réalisation technique :

### - Mélange et granulation :

Dans un mélangeur de type planétaire introduire la totalité de la cellulose.

Faire tourner le mélangeur à faible vitesse (vitesse 1) et incorporer progressivement de manière continue, la totalité de la solution extractive végétale.

Poursuivre l'agitation pour homogénéiser l'ensemble pendant cinq minutes.

### - Extrusion :

La masse humide est introduite dans l'extrudeur bivis axial, muni d'une filière d'orifice de 800 microns de diamètre moyen, d'une épaisseur de 800 microns.

La vitesse d'extrusion est de 100 t/mn.

La durée d'extrusion pour une masse de 8.550 g de cellulose microcristalline granulée humide est de 15 minutes à la température inférieure à 30°C (système d'extrusion tempéré entre 25° et 30°C).

Recueillir les extrudats dans un conteneur.

### - Sphéronisation :

La totalité des extrudats est introduite dans un sphéroniseur de 60 cm de diamètre.

La sphéronisation est engagée selon quatre cycles de vitesses et durées variables, pilotée automatiquement par informatique.
. cycle 1 : 740 t/mn - 180 secondes
. cycle 2 : 740 t/mn - 180 secondes
. cycle 3 : 740 t/mn - 180 secondes
. cycle 4 : 500 t/mn - 15 secondes

Recueillir les sphéroïdes dans un conteneur.

### - Séchage :

La totalité des sphéroïdes est introduite dans une turbine rotative munie d'un dispositif d'apport d'air chaud (40°C) avec aspiration de l'ambiance.

La turbine est mise en marche à faible vitesse (Vitesse 1), l'air chaud est orienté tangentiellement au lit de sphéroïdes.

Laisser en fonctionnement durant une heure.

Contrôler par pesée la teneur résiduelle en humidité. Lorsque cette teneur est inférieure à 5 % (m/m) de la masse, arrêter le séchage. Dans le cas contraire, maintenir l'apport d'air chaud par paliers de 10 minutes jusqu'à une teneur résiduelle en humidité inférieure à 5 % (m/m).

### - Calibrage :

Les sphéroïdes secs sont passés sur un dispositif automatique de calibrage comportant deux grilles de 1,000 mm et de 0,350 mm de diamètre de maille.

Les sphéroïdes compris entre ces deux tamis sont conditionnés en poche de polyéthylène qualité alimentaire, fermée et étiquetée avec le nom de la forme galénique, le nom de la drogue végétale, le numéro de lot.

### - Contrôle :

Le rendement granulométrique des sphéroïdes calibrés est de 97 %. La teneur en composants hydro alcoolo solubles est de 17,0 g pour 100 g de sphéroïdes. La teneur en harpagoside composant caractéristique est de 1,15 g pour 100 g de sphéroïdes.

### EXEMPLE 2

On a préparé de la façon décrite ci-dessus une forme de sphéroïdes contenant 11,7 % de constituants hydro alcoolo solubles de PASSIFLORE, correspondant à 100,0 parties de plante sèche et ayant la composition suivante.
pour un lot de 5.600 g environ des sphéroïdes la formule comporte :

| Ingrédient | Quantité en % en poids |
|---|---|
| Extrait liquide de PASSIFLORE | 5.100,0 g |
| Cellulose microcristalline | 5.000,0 g |

### Remarque :

L'extrait liquide contient 11,7 % de composants hydro alcoolo solubles de PASSIFLORE. Son titre alcoolique est de 45 %. Sa teneur en hétérosides exprimés en vitexine composant caractéristique est de 0,85 g % grammes d'extrait liquide.

On répète l'exemple 1 pour la réalisation technique.

### - Contrôle :

Le rendement granulométrique des sphéroïdes calibrés est de 97 %. La teneur en composants hydro alcoolo solubles est de 10,65 g pour 100 g de sphéroïdes. La teneur en hétérosides exprimés en vitexine composant caractéristique est de 0,77 g pour 100 g de sphéroïdes.

### EXEMPLE 3

On a préparé de la façon décrite ci-dessus une forme de sphéroïdes contenant 4,7 % de constituants hydro alcoolo solubles de GINSENG, 0,65 % pour l'ELEUTHERROCOQUE correspondant à 100,0 parties de plantes sèches et ayant la composition suivante.

Pour un lot de 5.650 g environ de sphéroïdes la formule comporte :

| Ingrédients | Quantité en % en poids |
|---|---|
| Extrait liquide de GINSENG | 1.275,0 g |
| Extrait d'ELEUTHEROCOQUE | 3.750,0 g |
| POLLEN | 27,5 g |
| Cellulose microcristalline | 5.000,0 g |

### Remarque :

L'extrait liquide contient 4,7 % de composants hydro alcoolo solubles de GINSENG, 0,65 pour l'ELEUTHEROCOQUE. Les titres alcooliques sont de 25 % dans les deux cas.

On répète l'exemple 1 pour la réalisation technique après avoir mélangé le POLLEN avec la cellulose microcristalline.

### - Contrôle :

Le rendement granulométrique des sphéroïdes calibrés est de 96,5 %. La teneur en composants hydro alcoolo solubles est de 1,65 g pour 100 g de sphéroïdes.

### EXEMPLE 4

On a préparé de la façon décrite ci-dessus une forme de sphéroïdes contenant 2,4 % de constituants hydro alcoolo solubles de VIGNE ROUGE correspondant à 100,0 parties de plante sèche et ayant la composition suivante.

Pour un lot de 5.100 g environ de sphéroïdes la formule comporte :

| Ingrédient | Quantité en % en poids |
|---|---|
| Extrait liquide de VIGNE ROUGE | 4.000,0 g |
| Lactose | 1.000,0 g |
| Cellulose microcristalline | 4.000,0 g |

### Remarque :

L'extrait liquide contient 2,4 % de composants hydro alcoolo solubles de VIGNE ROUGE. Son titre alcoolique est de 45 %. Sa teneur en flavonoïdes exprimés en malvidine composant caractéristique est de 0,3 g pour 100 g d'extrait liquide.

### Réalisation technique :

### - Contrôle :

Le rendement granulométrique des sphéroïdes calibrés est de 95,6 %. La teneur en composants hydro alcoolo solubles est de 1,88 g pour 100 g de sphéroïdes. La teneur en flavonoïdes exprimés en malvidine composant caractéristique est de 0,23 g pour 100 g de sphéroïdes.

### EXEMPLE 5

On a préparé de la façon décrite ci-dessus une forme de sphéroïdes contenant 0,0489 % de gluconate de zinc.

Pour un lot de 5.000 g environ de sphéroïdes la formule comporte :

| | |
|---|---|
| Solution de sel de zinc | 5.400,0 g |
| Cellulose microcristalline | 5.000,0 g |

### EXEMPLE 6

On a préparé de la façon décrite ci-dessus une forme de sphéroïdes contenant du béta caroténoïde, apporté par du jus de carotte.

Pour un lot de 5.000 g environ de sphéroïdes la formule comporte :

| | |
|---|---|
| Jus de carotte | 5.200,0 g |
| Cellulose microcristalline | 5.000,0 g |

### EXEMPLE 7

On a préparé de la façon décrite ci-dessus une forme de sphéroïdes contenant des éléments minéraux apportés par l'eau de mer.

Pour un lot de 5.000 g environ de sphéroïdes la formule comporte :

| | |
|---|---|
| Eau de mer partiellement désodée | 5.200,0 g |
| Cellulose microcristalline | 5.000,0 g |

### Remarque :

L'eau de mer peut contenir des quantités de composants solubles voisines de 30 à 40 %.

## Revendications

1. Procédé de production industrielle de sphéroïdes absorbables par voie orale dans lequel on prépare un extrait liquide de principes actifs, on met en contact cet extrait liquide avec un support absorbant et on sèche caractérisé en ce que l'on :
. prépare à partir d'une ou de plusieurs matières premières naturelles contenant au moins un principe actif un extrait liquide, contenant au moins le principe actif que l'on veut isoler par extraction aqueuse, alcoolique ou hydroalcoolique ou mixte ;
. humidifie une substance absorbante et adsorbante ou un mélange de ce type de substances par l'extrait liquide de telle sorte que la quantité d'extrait liquide soit comprise entre 50 et 250 % de la masse à humidifier ;
. extrude cette masse humide à une température inférieure à 30°C à travers une microfilière dont le rapport entre le diamètre des orifices et leur profondeur est sensiblement égal à l'unité pour obtenir des extrudats filaires ;
. sphéronise les extrudats filaires pour obtenir des sphéroïdes ;
. sèche les sphéroïdes ainsi obtenus à une température inférieure à 40°C ;
. calibre les sphéroïdes secs.

2. Procédé selon la revendication 1, caractérisé en ce que l'extrait liquide est une solution extractive d'une matière végétale.

3. Procédé selon la revendication 2, caractérisé en ce que l'extrait liquide est une solution extractive d'une matière végétale obtenue à partir d'une ou de plusieurs plantes.

4. Procédé selon la revendication 1, caractérisé en ce que l'on incorpore l'extrait liquide à une substance de type polymérique à fort pouvoir absorbant et adsorbant par mélange et homogénéisation.

5. Procédé selon la revendication 1, caractérisé en ce que la microfilière possède des orifices calibrés de diamètre et de profondeur de même dimension permettant une granulométrie comprise entre 350 et 1.000 microns.

6. Procédé selon la revendication 4, caractérisé en ce que les substances polymériques sont des polymères naturels du type dérivés cellulosiques, celluloses microcristallines ou amidons ou polysaccharides sensiblement égal en poids à celui de l'extrait liquide.

7. Procédé selon la revendication 4, caractérisé en ce que les substances polymériques sont mélangées à au moins une substance auxiliaire organique et/ou minérale.

8. Procédé selon la revendication 7, caractérisé en ce que les substances auxiliaires sont des sucres à faible pouvoir énergétique, des matières organiques et/ou minérales.

9. Procédé selon la revendication 1, caractérisé en ce que l'opération de sphéronisation s'effectue dans un sphéroniseur rotatif comprenant quatre phases de vitesse de durée variable.

10. Procédé selon la revendication 4, caractérisé en ce que la quantité de la solution d'extraits est comprise entre 50 % et 250 % en poids des substances de type polymérique utilisées pour la préparation.

11. Procédé selon la revendication 7, caractérisé en ce que les substances auxiliaires sont choisies parmi le lactose, le sorbitol, le mannitol, les malto-dextrines, les dérivés cellulosiques éthylé/méthylé/carboxylé, les gélatines à pouvoir gélifiant variable, la polyvinylpyrrolidone, les carbonates, les citrates.

12. Procédé selon la revendication 7, caractérisé en ce que les substances auxiliaires contiennent une cellulose microcristalline pour 20 % à 80 % de la masse totale à humidifier, du lactose pour 80 % à 20 %, de la gélatine à pouvoir gélifiant variable ou de la polyvinylpyrrolidone ou un dérivé cellulosique pour moins de 10 %.

13. Sphéroïdes susceptibles d'être obtenus par la mise en oeuvre du procédé selon les revendications de 1 à 12, caractérises en ce qu'ils résultent d'une formulation utilisant des solutions d'extraits de produits naturels incorporées à des substances de type polymérique absorbantes et adsorbantes préparées par un procédé d'extrusion et de sphéronisation.

14. Sphéroïdes selon la revendication 13, caractérisés en ce qu'ils contiennent jusqu'à 30 % de composants végétaux hydro alcoolo solubles.

## Claims

1. Process for industrial production of spheroids that can be absorbed orally, wherein a liquid extract of active principles is prepared, this liquid extract is contacted with an absorbent support and then dried, characterized in that:
- a liquid extract is prepared from one or more natural raw materials containing at least one active principle, where said liquid extract contains at least the active principle which it is desired to isolate by aqueous, alcoholic or hydroalcoholic, or mixed extraction;
- an absorbent and adsorbent substance or a mixture of this type of substances is wetted with the liquid extract in such a way that the quantity of liquid extract is between 50 and 250 % of the mass to be wetted;
- this moist mass is extruded at a temperature below 30°C through a micro-die of which the ratio between the diameter of the apertures and the depth thereof is substantially the same as the unit for obtaining filar extrudates;
- the filar extrudates are spheronised in order to obtain spheroids;
- the spheroids thereby obtained are dried at a temperature below 40°C;
- the dry spheroids are calibrated.

2. Process according to claim 1, characterised in that the liquid extract is a solution extractible from a vegetable material.

3. Process according to claim 2, characterized in that the liquid extract is a solution extractable from a vegetable material obtained from one or more plants.

4. Process according to claim 1, characterized in that the liquid extract is incorporated by mixing and homogenising into a substance of polymeric type having a high absorbing and adsorbing power.

5. Process according to claim 1, characterized in that the micro-die has calibrated apertures with a diameter and depth of the same size, making possible a particle size of between 350 and 1,000 microns.

6. Process according to claim 4, characterized in that the polymeric substances are natural polymers of the type comprising cellulose derivatives, microcrystalline celluloses or starches or polysaccharides, substantially having the same weight as the liquid extract

7. Process according to claim 4, characterized in that the polymeric substances are mixed with at least one organic and/or mineral auxiliary.

8. Process according to claim 7, characterized in that the auxiliaries are low-energy sugars, organic and/or mineral materials.

9. Process according to claim 1, characterized in that the spheronisation operation is carried out in a rotary spheroniser with four speed phases of variable duration.

10. Process according to claim 4, characterized in that the quantity of extract solution is between 50% and 250% by weight of the polymeric type substances used for the preparation.

11. Process according to claim 7, characterized in that the auxiliaries are selected from the group comprising lactose, sorbitol, mannitol, maltodextrins, ethylated/methylated/carboxylated cellulose derivatives, gelatins having variable gelling power, polyvinyl pyrrolidone, carbonates and citrates.

12. Process according to claim 7, characterized in that the auxiliaries comprise, based on the total mass to be wetted, 20% to 80% microcrystalline cellulose, 80% to 20% lactose, and less than 10% gelatin with variable gelling power or polyvinyl pyrrolidone or a cellulose derivative.

13. Spheroids obtainable by carrying out the process according to claims 1 to 12, characterized in that they are the result of a formulation utilizing solutions of extracts of natural products incorporated in absorbent and adsorbent substances of polymeric type prepared by a process of extrusion and spheronisation.

14. Spheroids according to claim 13, characterized in that they contain up to 30% soluble hydroalcoholic vegetable constituents.

## Patentansprüche

1. Verfahren zur industriellen Herstellung von Sphäroiden, zur oralen Verabreichung, indem ein flüssiger Extrakt von Wirkstoffkomponenten hergestellt, dieser mit einem absorbierenden Trägerstoff in Kontakt gebracht und getrocknet wird, gekennzeichnet durch folgende Schritte:
- Herstellen eines flüssigen Extrakts aus einem oder mehreren natürlichen Ausgangsstoffen mit wenigstens einer Wirkkomponente und mit wenigstens der durch Extraktion mit Wasser, Alkohol, wässriger alkoholischer Lösungen oder Mischungen hiervon zu extrahierende Wirkstoffkomponente;
- Befeuchten einer absorbierenden oder adsorbierenden Substanz oder einer Mischung derartiger Substanzen mit dem flüssigen Extrakt, so daß die Menge an flüssigem Extrakt zwischen 50 und 20 Mass.-% der zu befeuchtenden Substanz beträgt;
- Extrudieren dieser befeuchteten Substanz bei einer Temperatur unter 30°C in einer Mikrospinndüse, deren Öffnungen etwa das gleiche Verhältnis von Durchmesser und Tiefe aufweisen wie die Einheit zum Erhalt fadenförmiger Extrudate,
- Formen der fadenförmigen Extrudate in eine sphärische Gestalt zum Erhalt der Sphäroide;
- Trocknen der auf diese Weise erhaltenen Sphäroide bei einer Temperatur unter 40°C;
- Kalibrieren der getrockneten Sphäroide

2. Verfahren nach Anspruch 1, dadurch gekennzeichnet, daß der flüssige Extrakt eine extrahierte Lösung eines pflanzlichen Materials ist.

3. Verfahren nach Anspruch 2, dadurch gekennzeichnet, daß der flüssige Extrakt eine extrahierte Lösung eines aus einer oder mehreren Pflanzen erhaltenen pflanzlichen Materials ist.

4. Verfahren nach Anspruch 1, dadurch gekennzeichnet, daß der flüssige Extrakt einer polymeren Substanz mit hohem Ab- und Adsorptionsvermögen beim Vermischen und Homogenisieren zugesetzt wird.

5. Verfahren nach Anspruch 1, dadurch gekennzeichnet, daß die Mikrospinndüse in Durchmesser und Tiefe gleich kalibrierte Öffnungen aufweist, deren Abmessungen eine Kornverteilung zwischen 350 und 1000 µm ermöglichen.

6. Verfahren nach Anspruch 4, dadurch gekennzeichnet, daß die polymeren Substanzen Naturpolymere, wie Cellulosederivate, mikrokristalline Cellulose, Stärke oder Polysaccharide, sind mit etwa dem gleichen Gewicht wie das des flüssigen Extrakts.

7. Verfahren nach Anspruch 4, dadurch gekennzeichnet, daß die polymeren Substanzen mit wenigstens einem organischen und/oder mineralischen Hilfsstoff vermischt werden.

8. Verfahren nach Anspruch 7, dadurch gekennzeichnet, daß die Hilfsstoffe Zucker mit geringem Energiegehalt, organische und/oder mineralische Materialien sind.

9. Verfahren nach Anspruch 1, dadurch gekennzeichnet, daß das Formen zu Sphäroiden in einer rotierbaren Vorrichtung zum Erhalt von Sphäroiden mit vier Geschwindigkeitsstufen veränderbarer Dauer durchgeführt wird.

10. Verfahren nach Anspruch 4, dadurch gekennzeichnet, daß die Extraktmenge zwischen 50 und 250 Mass.-% bezogen auf die polymeren Substanzen bei der Präparation beträgt.

11. Verfahren nach Anspruch 7, dadurch gekennzeichnet, daß die Hilfsstoffe aus Lactose, Sorbit, Mannit, Maltodextrinen, Ethyl-/Methyl-/Carboxylcellulose-derivaten, Gelatine mit variablen Geliervermögen, Polyvinylpyrrolidon, Carbonaten und Citraten gewählt werden.

12. Verfahren nach Anspruch 7, dadurch gekennzeichnet, daß die Hilfsstoffe zwischen 20 und 80 Mass.-% mikrokristalline Cellulose, zwischen 80 und 20 Mass-% Lactose, und Gelatine mit variablem Geliervermögen oder Polyvinylpyrrolidon oder Cellulosederivate unter 10 Mass.-% bezogen auf die gesamte zu befeuchtende Masse enthalten.

13. Sphäroide, herstellbar mit einem Verfahren gemäß einem der Ansprüche 1 bis 12, dadurch gekennzeichnet, daß sie aus einer Formulierung von Extraktlösungen aus Naturprodukten, die polymeren Substanzen mit hohem Ab- und Adsorptionsvermögen zugesetzt sind, mittels eines Extrusions- und eines Formprozesses in eine sphärischen Form gebracht sind.

14. Sphäroide nach Anspruch 13, dadurch gekennzeichnet, daß sie bis zu 30% pflanzliche, in wässrigen alkoholischen Lösungen lösliche Komponenten enthalten.
